# EUROPÄISCHE PATENTSCHRIFT

(11) **EP 1 084 115 B1**
(45) Veröffentlichungstag und Bekanntmachung des Hinweises auf die Patenterteilung: **09.04.2003**
(21) Anmeldenummer: 99953337.5
(22) Anmeldetag: 10.05.1999
(51) Int. Cl.: C07D 307/89

(54) **VERFAHREN ZUR HERSTELLUNG VON PHTHALSÄUREANHYDRID**
METHOD FOR PRODUCING PHTHALIC ANHYDRIDE
PROCEDE DE PRODUCTION D'ANHYDRIDE PHTALIQUE

(30) Priorität: 26.05.1998 DE 19823262
(43) Veröffentlichungstag der Anmeldung: 21.03.2001
(73) Patentinhaber: BASF AKTIENGESELLSCHAFT, 67056 Ludwigshafen (DE)
(72) Erfinder: HEIDEMANN, Thomas, D-69469 Weinheim (DE); WANJEK, Herbert, D-67133 Maxdorf (DE)
(86) Internationale Anmeldenummer: EP9903191
(87) Internationale Veröffentlichungsnummer: WO99061433

(56) Entgegenhaltungen:
- EP-A- 0 522 871
- EP-A- 0 539 878
- EP-A- 0 744 214
- US-A- 4 046 780

## Beschreibung

Die Erfindung betrifft ein Verfahren zur Herstellung von Phthalsäureanhydrid, bei dem die katalytische Gasphasenoxidation von o-Xylol und/oder Naphthalin über mindestens 3 Schichten von Katalysatoren zunehmender Aktivität erfolgt und wobei die Zunahme der Aktivität der Schichten in bestimmter Weise bewirkt wird.

Bekanntermaßen wird eine Vielzahl von Carbonsäuren und/oder Carbonsäureanhydriden technisch durch die katalytische Gasphasenoxidation von aromatischen Kohlenwasserstoffen wie Benzol, den Xylolen, Naphthalin, Toluol oder Durol in Festbettreaktoren, vorzugsweise Rohrbündelreaktoren, hergestellt. Dabei werden beispielsweise Benzoesäure, Maleinsäureanhydrid, Phthalsäureanhydrid, Isophthalsäure, Terephthalsäure oder Pyromellithsäureanhydrid gewonnen. Dazu wird im Allgemeinen ein Gemisch aus einem molekularen Sauerstoff enthaltenden Gas, beispielsweise Luft und das zu oxidierende Ausgangsmaterial durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise einer Salzschmelze, umgeben. Trotz dieser Thermostatisierung kann es in der Katalysatorschüttung zur Ausbildung sogenannter "Heißer Flecken" (hot spots) kommen, in denen eine höhere Temperatur herrscht als im übrigen Teil der Katalysatorschüttung. Diese "hot spots" geben Anlaß zu Nebenreaktionen, wie der Totalverbrennung des Ausgangsmaterials oder führen zur Bildung unerwünschter, vom Reaktionsprodukt nicht oder nur mit viel Aufwand abtrennbarer Nebenprodukte, beispielsweise zur Bildung von Phthalid oder Benzoesäure bei der Herstellung von Phthalsäureanhydrid (PSA) aus o-Xylol. Des weiteren verhindert die Ausbildung eines ausgeprägten hot spots eine schnelle Hochfahrphase, da ab einer bestimmten hot spot-Temperatur der Katalysator irreversibel geschädigt werden kann, so dass die Beladungserhöhung nur in kleinen Schritten durchführbar ist und sehr sorgfältig kontrolliert werden muß (im weiteren als "Hochfahrphase" bezeichnet).

Zur Abschwächung dieser "hot spots" wurde in der Technik dazu übergegangen, unterschiedlich aktive Katalysatoren schichtweise in der Katalysatorschüttung anzuordnen, wobei in der Regel der weniger aktive Katalysator so im Festbett angeordnet ist, dass das Reaktionsgasgemisch mit ihm als erstes in Kontakt kommt, d.h. er liegt in der Schüttung zum Gaseintritt hin, wohingegen sich der aktivere Katalysator zum Gasaustritt aus der Katalysatorschüttung hin befindet (DE-OS 2546268, EP 286 448, DE 2948163, EP 163 231). Die unterschiedlich aktiven Katalysatoren in der Katalysatorschüttung können bei der gleichen Temperatur dem Reaktionsgas ausgesetzt werden, es können die beiden Schichten aus unterschiedlich aktiven Katalysatoren aber auch auf unterschiedliche Reaktionstemperaturen thermostatisiert mit dem Reaktionsgas in Kontakt gebracht werden (DE-OS 2830765). Nach EP 163 231 können mehrere der genannten Maßnahmen zur Einstellung der beschriebenen Aktivitätsstrukturierung gleichzeitig angewendet werden. Aus WO 98/00778 ist bekannt, dass der Zusatz temporärer Aktivitätsdämpfungsmittel zu einer Verkürzung der Hochfahrphase führen kann. Des weiteren wird in EP 676 400 durch Mehrfachstrukturierung bei der Umsetzung von Tetraalkylbenzolen zu Pyromellithsäureanhydrid ein positiver Effekt bzgl. Ausbeute und Produktreinheit festgestellt, wenn man die Aktivitätsstrukturierung so vornimmt, dass die Katalysatoraktivität in Strömungsrichtung des Gases zunächst zu- und anschließend wieder abnimmt. Schließlich wird in EP 99 431 bei der Umsetzung von Butan zu Maleinsäureanhydrid beschrieben, dass durch eine Aktivitätsstrukturierung des Katalysatorbettes ein positiver Effekt bzgl. der Produktivität dann vorliegt, wenn die Katalysatoraktivität in Strömungsrichtung des Gases stufenweise (bzw. im Idealfall kontinuierlich) zunimmt, wobei die Aktivitätsstrukturierung durch eine Vielzahl unterschiedlicher Methoden erreicht werden kann, bevorzugt durch Verdünnung mittels Inertmaterial. Durch die stufenweise Aktivitätszunahme kann eine homogenere Verteilung der freiwerdenden Energie der exothermen Reaktion erreicht werden, so dass größere Mengen MSA hergestellt werden können. Da die Reaktion unter Teilumsatz durchgeführt wird, ist die Art und Weise, wie die Aktivitässtrukturierung erreicht wird, nahezu beliebig. Diese Lehre ist nicht auf die PSA-Herstellung mittels o-Xylolund/oder Naphthalinoxidation übertragbar, da wie bekannt nur dann ein Phthalsäureanhydrid guter Qualität erhalten wird, wenn die Reaktion unter Vollumsatz (d.h. > 99,9 % Umsatz bzgl. des eingesetzten Eduktes) ausgeführt wird, um Verunreinigungen an störenden farbgebenden Komponenten wie Phthalid bzw. Naphthochion zu minimieren und um Verunreinigungen des Abgases durch Rest-Xylol bzw. Rest-Naphthalin zu vermeiden.

Aus der EP-A 539 878 ist ein Verfahren zur Herstellung von Phthalsäureanhydrid aus o-Xylol und Naphthalin an einem zwei-Schichten-Katalysator bekannt. Die Katalysatoren in beiden Schichten sind so aufgebaut, dass ein inaktiver Träger mit Vanadiumpentoxid, Anatas-Titandioxid sowie Niob, Phosphor, Antimon und mindestens einem Elementoxid von Kalium, Rubidium, Cäsium Thallium belegt ist, wobei sich der Katalysator der zweiten Schicht durch seinen geringeren Gehalt an dem mindestens einen Elementoxid von Kalium, Rubidium, Cäsium und Thallium von dem Katalysator der ersten Schicht unterscheidet.

Trotz der vorgenannten Verbesserungsvorschläge sind lange Hochfahrzeiten von 2-8 Wochen oder länger bisher erforderlich. "Hochfahrzeit" beschreibt die Zeit, die notwendig ist, um den Katalysator auf die gewünschte Endbeladung, nach gängigem Stand der Technik 80 g o-Xylol/Nm³-Luft oder höher, d.h., die Oxidation zum stationären Zustand zu bringen, ohne den Katalysator irreversibel zu schädigen. Hierbei ist vor allem darauf zu achten, dass der hot spot einen gewissen kritischen Wert nicht überschreitet (d.i. üblicherweise 450-480°C), da sonst die PSA-Selektivität, die PSA-Produktqualität sowie die Lebensdauer des Katalysators stark beeinträchtigt werden.

Es bestand daher die Aufgabe, ein Verfahren zur Herstellung von Phthalsäureanhydrid zu finden, bei dem es durch Auswahlkombination von bestimmter Katalysatorschichtung gelingt, alle gewünschten Parameter wie kurze Hochfahrzeit, hohe Ausbeute und geringe Bildung von Nebenprodukten sowie gute Produktqualität wie einen geringeren Phthalidgehalt, zu vereinen.

Diese Aufgabe wurde erfindungsgemäß gelöst mit einem Verfahren zur Herstellung von Phthalsäureanhydrid durch katalytische Gasphasenoxidation von Xylol und/oder Naphthalin mit einem molekularen Sauerstoff enthaltenden Gas in einem Festbett bei erhöhter Temperatur und mittels mindestens drei in Schichten übereinander angeordneter Schalenkatalysatoren, auf deren Kern aus Trägermaterial eine Schicht aus katalytisch aktiven Metalloxiden schalenförmig aufgebracht ist, bei dem die Katalysatoraktivität von Schicht zu Schicht von der Gaseintrittsseite zur Gasaustrittsseite ansteigt, wobei die Aktivität der Katalysatoren der einzelnen Schichten so eingestellt wird, dass der am geringsten aktive Katalysator eine geringere Aktivmassengehalt und gegebenenfalls zusätzlich mehr Alkalimetall, ausgewählt aus der Gruppe bestehend aus Kalium, Rubidium und Cäsium, als Dotierung als der Katalysator der nächsten Schicht aufweist und der darauf folgende noch aktivere Katalysator die gleiche Aktivmassenmenge und noch weniger Alkalimetall als Dotierung oder eine größere Aktivmassenmenge und gegebenenfalls weniger Alkalimetall als Dotierung als der Katalysator der zweiten Schicht aufweist, mit der Maßgabe, dass
a) der am geringsten aktive Katalysator auf nicht porösem Trägermaterial 5 bis 9 Gew.-%, bezogen auf den gesamten Katalysator, Aktivmasse aufweist, enthaltend 3 bis 8 Gew.-% V₂O₅, 0 bis 3,5 Gew.-% Sb₂O₃, 0 bis 0,3 Gew.-% P, 0,1 bis 0,5 Gew.-% Alkali (ber. als Alkalimetall) und als Rest TiO₂ in Anatasform mit einer BET-Oberfläche (J. Amer. Chem. Soc. Band 60, Seite 309 ff. (1938)) von 18 bis 22 m²/g,
b) der nächst aktivere Katalysator bei sonst gleicher Zusammensetzung wie Katalysator (a) einen um 1 bis 5 Gew.-% (absolut) höheren Aktivmassengehalt hat und der Alkaligehalt um 0 bis 0,25 Gew.-% (absolut) geringer ist und
c) der aktivste Katalysator bei sonst gleicher Zusammensetzung wie (a) einen um 1 bis 5 Gew.-% (absolut) höheren Aktivmassengehalt als für (a) hat und der Alkaligehalt um 0,15 bis 0,4 Gew.-% (absolut) geringer als für (a) ist.

Demgemäß hat der Katalysator (a), der der Gaseintrittsseite am nächsten ist, immer eine geringere Menge Aktivmasse (und gegebenenfalls zusätzlich einen die Aktivität mindernden Zusatz von Alkali, insbesondere Cäsium) als der folgende Katalysator (b), der wiederum eine geringere oder die gleiche Menge Aktivmasse wie der darauffolgende Katalysator (c) haben kann, wobei im Falle der gleichen Menge Aktivmasse der Alkaligehalt größer als der des Katalysators (c) sein muß. Unter der Maßgabe dieser Bedingungen weist der Katalysator (a) nach einer bevorzugten Ausführungsform einen Aktivmassengehalt von 6 bis 8 Gew.-% auf, enthaltend 4 bis 8 Gew.-% V₂O₅ und 0,3 bis 0,5 Gew.-% Cs (berechnet als Cs), jeweils bezogen auf die Aktivmasse, der Katalysator (b) einen Aktivmassengehalt von 8 bis 12 Gew.-%, enthaltend 0,2 bis 0,5 Gew.-% Cs bezogen auf die Aktivmasse und der Katalysator (c) einen Aktivmassengehalt von 8 bis 12 Gew.-%, enthaltend 0 bis 0,3 Gew.-% Cs bezogen auf die Aktivmasse . Nach der besonders bevorzugten Arbeitsweise hat der Katalysator (a) einen Aktivmassengehalt von 7 bis 8 Gew.-%, enthaltend 6 bis 8 Gew.-% V₂O₅ und 0,3 bis 0,4 Gew.-% Cs, jeweils bezogen auf die Aktivmasse, der Katalysator (b) einen Aktivmassengehalt von 9 bis 11 Gew.-%, enthaltend 0,2 bis 0,4 Gew.-% Cs bezogen auf die Aktivmasse und der Katalysator (c) einen Aktivmassengehalt von 9 bis 11 Gew.-%, enthaltend 0,05 bis 0,2 Gew.-% Cs (jeweils berechnet als Cs) bezogen auf die Aktivmasse.

Anstelle von gegeneinander abgegrenzten Schichten der verschiedenen Katalysatoren kann auch ein quasi-kontinuierlicher Übergang der Schichten und ein quasi-gleichmässiger Anstieg der Aktivität dadurch bewirkt werden, dass man beim Übergang von einer Schicht zur nächsten Schicht eine Zone mit einer Vermischung der aufeinander folgenden Katalysatoren vornimmt.

Die für die verschiedenen Schichten verwendeten Katalysatoren (a), (b) und (c) sind an sich bekannt und deren Herstellung und Zusammensetzung ist vielfach beschrieben. Es handelt sich, kurz dargestellt, bei diesen Katalysatoren in der Regel um sogenannte Schalenkatalysatoren, bei denen die katalytisch aktive Masse üblicherweise schalenförmig auf einem im Allgemeinen unter den Reaktionsbedingungen inerten, nicht porösen Träger material- wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im Allgemeinen neben Titandioxid in Form seiner Anatasmodifikation Vanadiumpentoxid. Des weiteren können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise in-dem sie seine Aktivität absenken oder erhöhen. Als solche Promotoren seien beispielhaft die Alkalimetalloxide, insbesondere Lithium-, Kalium-, Rubidium- und Cäsiumoxid, Thallium-(I)-oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Als die Aktivität vermindernder und die Selektivität erhöhender Promotor wirken z.B. die Alkalimetalloxide, wohingegen oxidische Phosphorverbindungen, insbesondere Phosphorpentoxid, die Aktivität des Katalysators erhöhen, aber dessen Selektivität vermindern.

Zur Herstellung derartiger Schalenkatalysatoren wird nach den Verfahren von DE-A 1642938 und DE-A 1769998 eine wäßrige und/oder ein organisches Lösungsmittel enthaltende Lösung oder Suspension der Aktivmassenbestandteile und/oder deren Vorläuferverbindungen, welche im folgenden als "Maische" bezeichnet wird, auf das Trägermaterial in einer beheizten Dragiertrommel bei erhöhter Temperatur aufgesprüht, bis der gewünschte Aktivmassenanteil am Katalysatorgesamtgewicht erreicht ist. Nach DE 2106796 läßt sich die Beschichtung auch in Wirbelbeschichtern durchführen, wie sie z.B. in DE 1280756 angegeben sind. Beim Aufsprühen in der Dragiertrommel sowie beim Beschichten im Wirbelbett treten allerdings hohe Verlust auf, da erhebliche Menge der Maische vernebelt bzw. durch Abrasion Teile der bereits aufbeschichteten Aktivmasse wieder abgerieben und durch das Abgas ausgetragen werden. Da der Aktivmasseanteil am Gesamtkatalysator im Allgemeinen nur eine geringe Abweichung vom Sollwert haben soll, da durch die Menge der aufgebrachten Aktivmasse und damit die Schichtdicke der Schale Aktivität und Selektivität des Katalysators stark beeinflußt werden, muß der Katalysator bei den geschilderten Herstellungsweisen häufig zur Bestimmung der aufgebrachten Aktivmassenmenge abgekühlt, aus der Dragiertrommel bzw. der Wirbelschicht entnommen und nachgewogen werden. Wird zuviel Aktivmasse auf dem Katalysatorträger abgeschieden, ist im Allgemeinen eine nachträgliche, schonende Entfernung der zuviel aufgetragenen Aktivmassemenge ohne Beeinträchtigung der Festigkeit der Schale, insbesondere,ohne Rißbildung in der Katalysatorschale, nicht möglich.

Um diese Problem abzumildern, wurde in der Technik dazu übergegangen, der Maische organische Binder, bevorzugt Copolymere, vorteilhaft in Form einer wäßrigen Dispersion, von Vinylacetat/Vinyllaurat, Vinylacetat/Acrylat, Styrol/Acrylat, Vinylacetat/Maleat sowie Vinylacetat/Ethylen zuzusetzen, wobei Bindermengen von 10 - 20 Gew.-%, bezogen auf den Feststoffgehalt der Maische, eingesetzt wurden (EP 744214). Wird die Maische ohne organische Bindemittel auf den Träger aufgetragen, so sind Beschichtungstemperaturen über 150°C von Vorteil. Bei Zusatz der oben angegebenen Bindemittel liegen die brauchbaren Beschichtungstemperaturen je nach verwendetem Bindemittel zwischen 50 und 450°C (DE 2106796). Die aufgetragenen Bindemittel brennen nach dem Einfüllen des Katalysators in den Reaktor und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

Weitere geeignete Verfahren zur Herstellung von Schalenkatalysatoren für die katalytische Gasphasenoxidation von aromatischen Kohlenwasserstoffen zu Carbonsäuren und/oder Carbonsäureanhydriden sind in WO 98-00778 bzw. EP-A 714700 beschrieben. Die katalytisch aktive Metalloxide enthaltende Schicht wird in der Form auf ein Trägermaterial aufgebracht, dass aus einer Lösung und/oder einer Suspension der katalytisch aktiven Metalloxide und/oder deren Vorläuferverbindungen, gegebenenfalls in Anwesenheit von Hilfsmitteln für die Katalysatorherstellung zunächst ein Pulver hergestellt wird, das anschließend für die Katalysatorherstellung auf dem Träger, gegebenenfalls nach Konditionierung sowie gegebenenfalls nach Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide oder einer Behandlung zur Entfernung flüchtiger Bestandteile unterzogen wird.

Die Bedingungen des Verfahrens zur Herstellung von Phthalsäureanhydrid aus o-Xylol und/oder Naphthalin sind gleichermaßen literaturbekannt. Insbesondere wird auf eine zusammenfassende Darstellung in K. Towae, W. Enke, R. Jäckh, N. Bhargana "Phtalic Acid and Derivatives" in Ullmann's Encyclopedia of Industrial Chemistry Vol. A. 20, 1992, 181 verwiesen und hiermit Bezug genommen. Abweichend von dem aus der Literaturstelle Bekannten gelingt es mit dem vorliegenden Verfahren, die "Hochfahrzeiten". d.h. die bis zum Erreichen des stationären Zustands erforderlichen Zeiten, auf in der Regel 5 bis 20 Tage zu verkürzen. Ansonsten gelten für den stationären Betriebszustand der Oxidation die aus dieser Literaturstelle und daneben etwa aus der WO-A 98/37967 bekannten Randbedingungen.

Dazu werden zunächst die Katalysatoren in die Reaktionsrohre des Reaktors, die von außen auf die Reaktionstemperatur, beispielsweise mittels Salzschmelzen thermostatisiert sind, gefüllt. Über die so bereitete Katalysatorschüttung wird das Reaktionsgas bei Temperaturen von im Allgemeinen 300 bis 450°C, vorzugsweise 320 bis 420 und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von im Allgemeinen 750 bis 5000 h⁻¹ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel wie Dampf, Kohlendioxid und/oder Stickstoff enthalten kann, mit dem zu oxidierenden, aromatischen Kohlenwasserstoff erzeugt, wobei das den molekularen Sauerstoff enthaltende Gas im Allgemeinen 1 bis 100 , vorzugsweise 2 bis 50 und besonders bevorzugt 10 bis 30 mol-% Sauerstoff, 0 bis 30, vorzugsweise 0 bis 10 mol-% Wasserdampf sowie 0 bis 50, vorzugsweise 0 bis 1 mol-% Kohlendioxid, Rest Stickstoff, enthalten kann. Zur Erzeugung des Reaktionsgases wird das den molekularen Sauerstoff enthaltende Gas im Allgemeinen mit 30 bis 150 g je Nm³ Gas des zu oxidierenden, aromatischen Kohlenwasserstoffs beschickt.

### Beispiele

Der in den folgenden Beispielen verwendete Anatas hatte typischerweise folgende Zusammensetzung: Neben TiO₂ 0,18 %Gew.-% S; 0,08 %Gew.-% P; 0,24 %Gew.-% Nb; 0,01 %Gew.-% Na; 0,01 %Gew.-% K; 0,004 %Gew.-% Zr; 0,004 %Gew.-% Pb.

### Beispiel 1: Herstellung des Katalysators I(a)

700 g Steatit (Magnesiumsilikat) in Form von Ringen mit einem äußeren Durchmesser von 8 mm, einer Länge von 6 mm und einer Wandstärke von 1,5 mm wurden in einer Dragiertrommel auf 210°C erhitzt und mit einer Suspension aus 400,0 g Anatas mit einer BET-Oberfläche von 21 m²/g, 57,6 g Vanadyloxalat, 14,4 g Antimontrioxid, 3,3 g Ammoniumhydrogenphosphat, 2,60 g Cäsiumsulfat, 618 g Wasser und 128 g Formamid besprüht, bis das Gewicht der aufgetragenen Schicht 7,1 % des Gesamtgewichtes des fertigen Katalysators betrug. Die auf diese Weise aufgebrachte katalytisch aktive Masse, also die Katalysatorschale, enthielt 0,2 Gew.-% Phosphor (berechnet als P), 7,5 Gew.-% Vanadium (berechnet als V₂O₅), 3,2 Gew.-% Antimon (berechnet als Sb₂O₃), 0,40 Gew.-% Cäsium (berechnet als Cs) und 88,75 Gew.-% Titandioxid.

### Beispiel 2: Herstellung des Katalysators II (a)

700 Steatit (Magnesiumsilikat) in Form von Ringen mit einem äußeren Durchmesser von 8 mm, einer Länge von 6 mm und einer Wandstärke von 1,5 mm wurde in einer Dragiertrommel auf 210°C erhitzt und mit einer Suspension aus 400,0 g Anatas mit einer BET-Oberfläche von 21 m²/g, 57,6 g Vanadyloxalat, 14,4 Antimontrioxid, 3,3 g Ammoniumhydrogenphosphat, 2,28 Cäsiumsulfat, 618 g Wasser und 128 g Formamid besprüht, bis das Gewicht der aufgetragenen Schicht 7,5 % des Gesamtgewichts des fertigen Katalysators betrug. Die auf diese Weise aufgebrachte katalytisch aktive Masse, also die Katalysatorschale, bestand aus 0,2 Gew.-% Phosphor (berechnet als P), 7,5 Gew.-% Vanadium (berechnet als V₂O₅), 3,2 Gew.-% Antimon (berechnet als Sb₂O₃), 0,35 Gew.-% Cäsium (berechnet als Cs) und 88,8 Gew.-% Titandioxid.

### Beispiel 3: Herstellung des Katalysators III

700 Steatit (Magnesiumsilikat) in Form von Ringen mit einem äußeren Durchmesser von 8 mm, einer Länge von 6 mm und einer Wandstärke von 1,5 mm wurde in einer Dragiertrommel auf 210°C erhitzt und mit einer Suspension aus 400,0 g Anatas mit einer BET-Oberfläche von 21 m²/g, 57,6 g Vanadyloxalat, 14,4 Antimontrioxid, 3,3 g Ammoniumhydrogenphosphat, 2,28 Cäsiumsulfat, 618 g Wasser und 128 g Formamid besprüht, bis das Gewicht der aufgetragenen Schicht 6,8 % des Gesamtgewichts des fertigen Katalysators betrug. Die auf diese Weise aufgebrachte katalytisch aktive Masse, also die Katalysatorschale, bestand aus 0,2 Gew.-% Phosphor (berechnet als P), 7,5 Gew.-% Vanadium (berechnet als V₂O₅), 3,2 Gew.-% Antimon (berechnet als Sb₂O₃), 0,35 Gew.-% Cäsium (berechnet als Cs) und 88,8 Gew.-% Titandioxid.

### Beispiel 4: Herstellung des Katalysators IV (b)

700 Steatit (Magnesiumsilikat) in Form von Ringen mit einem äußeren Durchmesser von 8 mm, einer Länge von 6 mm und einer Wandstärke von 1,5 mm wurde in einer Dragiertrommel auf 210°C erhitzt und mit einer Suspension aus 400,0 g Anatas mit einer BET-Oberfläche von 21 m²/g, 57,6 g Vanadyloxalat, 14,4 Antimontrioxid, 3,3 g Amoniumhydrogenphosphat, 2,28 Cäsiumsulfat, 618 g Wasser und 128 g Formamid besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand aus 0,2 Gew.-% Phosphor (berechnet als P), 7,5 Gew.-% Vanadium (berechnet als V₂O₅), 3,2 Gew.-% Antimon (berechnet als Sb₂O₃), 0,40 Gew.-% Cäsium (berechnet als Cs) und 88,75 Gew.-% Titandioxid.

### Beispiel 5: Herstellung des Katalysators V (b)

700 Steatit (Magnesiumsilikat) in Form von Ringen mit einem äußeren Durchmesser von 8 mm, einer Länge von 6 mm und einer Wandstärke von 1,5 mm wurde in einer Dragiertrommel auf 210°C erhitzt und mit einer Suspension aus 400,0 g Anatas mit einer BET-Oberfläche von 21 m²/g, 57,6 g Vanadyloxalat, 14,4 Antimontrioxid, 3,3 g Amoniumhydrogenphosphat, 2,28 Cäsiumsulfat, 618 g Wasser und 128 g Formamid besprüht, bis das Gewicht der aufgetragenen Schicht 10,1 % des Gesamtgewichts des fertigen Katalysators betrug. Die auf diese Weise aufgebrachte katalytisch aktive Masse, also die Katalysatorschale, bestand aus 0,2 Gew.-% Phosphor (berechnet als P), 7,5 Gew.-% Vanadium (berechnet als V₂O₅), 3,2 Gew.-% Antimon (berechnet als Sb₂O₃), 0,35 Gew.-% Cäsium (berechnet als Cs) und 88,8 Gew.-% Titandioxid.

### Beispiel 6: Herstellung des Katalysator VI

700 Steatit (Magnesiumsilikat) in Form von Ringen mit einem äußeren Durchmesser von 8 mm, einer Länge von 6 mm und einer Wandstärke von 1,5 mm wurde in einer Dragiertrommel auf 210°C erhitzt und mit einer Suspension aus 400,0 g Anatas mit einer BET-Oberfläche von 21 m²/g, 57,6 g Vanadyloxalat, 14,4 Antimontrioxid, 3,3 g Amoniumhydrogenphosphat, 2,28 Cäsiumsulfat, 618 g Wasser und 128 g Formamid besprüht, bis das Gewicht der aufgetragenen Schicht 10,6 % des Gesamtgewichts des fertigen Katalysators betrug. Die auf diese Weise aufgebrachte katalytisch aktive Masse, also die Katalysatorschale, bestand aus 0,2 Gew.-% Phosphor (berechnet als P), 7,5 Gew.-% Vanadium (berechnet als V₂O₅), 3,2 Gew.-% Antimon (berechnet als Sb₂O₃), 0,30 Gew.-% Cäsium (berechnet als Cs) und 88,85 Gew.-% Titandioxid.

### Beispiel 7: Herstellung des Katalysators VII (c)

700 Steatit (Magnesiumsilikat) in Form von Ringen mit einem äußeren Durchmesser von 8 mm, einer Länge von 6 mm und einer Wandstärke von 1,5 mm wurde in einer Dragiertrommel auf 210°C erhitzt und mit einer Suspension aus 400,0 g Anatas mit einer BET-Oberfläche von 21 m²/g, 57,6 g Vanadyloxalat, 14,4 Antimontrioxid, 3,3 g Amoniumhydrogenphosphat, 2,28 Cäsiumsulfat, 618 g Wasser und 128 g Formamid besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand aus 0,2 Gew.-% Phosphor (berechnet als P), 7,5 Gew.-% Vanadium (berechnet als V₂O₅), 3,2 Gew.-% Antimon (berechnet als Sb₂O₃), 0,10 Gew.-% Cäsium (berechnet als Cs) und 89,05 Gew.-% Titandioxid.

### Beispiel 8: Herstellung von PSA bei Beladung bis 85 g o-Xylol/Nm³-Luft (erfindungsgemäß)

1,00 m des Katalysators I(a) , 0,60 m des Katalysators IV (b) und 1,30 m des Katalysators VII (c) wurden in ein 3,85 m langes Eisenrohr mit einer lichten Weite von 25 mm eingefüllt. Das Eisenrohr war zur Temperaturregelung von einer Salzschmelze umgeben, eine 4 mm Thermohülse mit eingebautem Zugelement diente der Katalysatortemperaturmessung. Durch das Rohr wurden stündlich von oben nach unten 4,0 Nm³-Luft mit Beladungen an 98,5 Gew.-%igem o-Xylol von 0 bis 85 g/Nm³ geleitet, dabei wurden bei 50-85 o-Xylol/Nm³ Luft die in Tabelle 1 zusammengefaßten Ergebnisse erhalten ("Ausbeute" bedeutet das erhaltene PSA in Gewichtsprozent, bezogen auf 100%iges o-Xylol; "Hochfahrzeit" bedeutet die zur Beladungserhöhung von 0 auf 80 g/nm³ benötigten Tage).

### Beispiel 9: Herstellung von PSA bei Beladungen bis 85 g o-Xylol/Nm³-Luft (erfindungsgemäß)

Man verfährt wie im Beispiel 8 beschrieben, mit dem Unterschied, dass die Katalysatoren II(a), V (b) und VII (c) eingesetzt werden.

### Beispiel 10: Herstellung von PSA bei Beladungen bis 85 g o-Xylol/Nm³-Luft (Vergleich)

1,60 m des Katalysators IV (b) und 1,30 m des Katalysators VII (c) wurden in ein 3,85 m langes Eisenrohr mit einer lichten Weite von 25 mm eingefüllt. Das Eisenrohr war zur Temperaturregelung von einer Salzschmelze umgeben, eine 4 mm Thermohülse mit eingebautem Zugelement diente der Katalysatortemperaturmessung. Durch das Rohr wurden stündlich von oben nach unten 4,0 Nm³-Luft mit Beladungen an 98,5 Gew.-%igem o-Xylol von 0 aussteigend bis etwa 85 g/Nm³-Luft geleitet. Dabei wurden bei 50-85 g o-Xylol/Nm³ Luft die in Tabelle 1 zusammengefaßten Ergebnisse erhalten ("Ausbeute" bedeutet das erhaltene PSA in Gewichtsprozent, bezogen auf 100 %iges o-Xylol, "Hochfahrzeit" bedeutet die zur Beladungserhöhung von 0 auf 80g/nm³ benötigten Tage).

### Beispiel 11: Herstellung von PSA bei Beladungen bis 85g o-Xylol/Nm³-Luft (Vergleich)

2,10 m des Katalysators Ia und 0,80 m des Katalysators VII (c) wurden in ein 3,85m langes Eisenrohr mit einer lichten Weite von 25 mm eingefüllt. Das Eisenrohr war zur Temperaturregelung von einer Salzschmelze umgeben, eine 4 mm Thermohülse mit eingebautem Zugelement diente der Katalysatortemperaturmessung. Durch das Rohr wurden stündlich von oben nach unten 4,0 Nm³-Luft mit Beladungen an 98,5 Gew.-%igem o-Xylol von 0 ansteigend bis etwa 85g/Nm³-Luft geleitet. Dabei wurden bei 50-85g o-Xylol/Nm³ Luft die in Tabelle 1 zusammengefaßten Ergebnisse erhalten ("Ausbeute" bedeutet das erhaltene PSA in Gewichtsprozent, bezogen auf 100 %iges o-Xylol; "Hochfahrzeit" bedeutet die zur Beladungserhöhung von 0 auf 80 g/Nm³ benötigten Tage.

### Beispiel 12: Herstellung von PSA bei Beladungen bis 85g o-Xylol/Nm³-Luft (Vergleich)

2,10 m eines Katalysators, bestehend aus einem Gemisch aus 75 Gew.-% des Katalysator IV (b) 25 Gew.-% Steatit (Magnesiumsilikat) in Form von Ringen (mit einem äußeren Durchmesser von 8mm, einer Länge von 6mm und einer Wandstärke von 1,5mm) und 0,80m des Katalysators VII (c) wurden in ein 3,85 m langes Eisenrohr mit einer lichten Weite von 25 mm eingefüllt. Das Eisenrohr war zur Temperaturregelung von einer Salzschmelze umgeben, eine 4 mm Thermohülse mit eingebauten Zugelement diente der Katalysatortemperaturmessung. Durch das Rohr wurden stündlich von oben nach unten 4,0Nm³-Luft mit Beladungen an 98,5 Gew.-% o-Xylol von 0 bis 85 g/Nm³-Luft geleitet. Dabei wurden bei 50-85 g o-Xylol/Nm³ Luft die in Tabelle 1 zusammengefaßten Ergebnisse erhalten ("Ausbeute" bedeutet das erhaltene PSA in Gewichtsprozent, bezogen auf 100 %iges o-Xylol; "Hochfahrzeit" bedeutet die zur Beladungserhöhung von 0 auf 80 g/Nm³ benötigten Tage.

### Beispiel 13: Herstellung von PSA bei Beladungen bis 85g o-Xylol/Nm³-Luft (Vergleich)

Man verfährt wie unter Beispiel 8 beschrieben, mit dem Unterschied, dass als Katalysator III, VI und VII (c) eingesetzt werden.

**Tabelle 1:**

| Beispiel: Katalysatorkombination | Salzbad-temperatur (°C) | Hochfahrzeit | durchschnittliche PSA-Ausbeute während 30d (Gew.-%) | durchschnittlicher Phthalidgehalt im Roh-PSA während 30d (Mol-%) |
|---|---|---|---|---|
| 8: I(a), IV (b), VII (c) | 380-370 | 7 | 111,5 | 0,10-0,19 |
| 9:II(a), V(b), VII (c) | 370-366 | 10 | 113 | 0,15-0,25 |
| 10:Vergleich IV(b)/VII(c ) | 365-355 | 32 | 112,5 | 0,05-0,22 |
| 11: Vergleich I(a)/VII(c) | 380-370 | 10 | 113 | 0,37-0,58 |
| 12:Vergleich IV (b) + Steatit/VII (c) | 375-365 | 11 | 113 | 0,33-0,55 |
| 13:Vergleich III/ VI/VIIc | 380-370 | Beladung kann auch über einen längeren Zeitraum nicht auf Werte über 40g o-Xylol/Nm³-Luft erhöht werden | (sieheAnmerkung bei " Hochfahrzeit") | (sieheAnmerkung bei "Hochfahrzeit") |

### Beispiel 14: Herstellung von PSA bei Beladungen bis 105g o-Xylol/Nm³-Luft (erfindungsgemäß)

Man verfährt wie unter Beispiel 8 beschrieben, mit dem Unterschied, dass durch das Rohr stündlich von oben nach unten 4,0 Nm³-Luft mit Beladungen an 98,5 Gew.-%igem o-Xylol von 85 bis etwa 105g/Nm³-Luft geleitet werden. Dabei wurden bei 95-105g o-Xylol/Nm³ Luft die in Tabelle 2 zusammengefaßten Ergebnisse erhalten ("Ausbeute" bedeutet das erhaltene PSA in Gewichtsprozent, bezogen auf 100%iges o-Xylol; "Lasterhöhungszeit" bedeutet die zur Beladungserhöhung von 80 auf 105g/Nm³ benötigten Tage).

### Beispiel 15: Herstellung von PSA bei Beladung bis 105g o-Xylol/Nm³-Luft (erfindungsgemäß)

Man verfährt wie unter Beispiel 9 beschrieben, mit dem Unterschied, dass durch das Rohr stündlich von oben nach unten 4,0 Nm³-Luft mit Beladungen an 98,5 Gew.-%igem o-Xylol von 85 bis etwa 105g/Nm³-Luft geleitet werden. Dabei wurden bei 95-105g o-Xylol/Nm³ die in Tabelle 2 zusammengefaßten Ergebnisse erhalten ("Ausbeute" bedeutet das erhaltene PSA in Gewichtsprozent, bezogen auf 100%iges o-Xylol; "Lasterhöhungszeit" bedeutet die zur Beladungserhöhung von 80 auf 105g/Nm³ benötigten Tage).

### Beispiel 16: Herstellung von PSA bei Beladungen bis 105g o-Xylol/Nm³-Luft (Vergleich)

Man verfährt wie unter Beispiel 11 beschrieben, mit dem Unterschied, dass durch das Rohr stündlich von oben nach unten 4,0 Nm³-Luft mit Beladungen an 98,5 Gew.-%igem o-Xylol von 85 auf etwa 105g/Nm³-Luft geleitet wird. Dabei wurden bei 95-105 g o-Xylol/Nm³ Luft die in Tabelle 2 zusammengefaßten Ergebnisse erhalten ("Ausbeute" bedeutet das erhaltene PSA in Gewichtsprozent, bezogen auf 100%iges o-Xylol; "Lasterhöhungszeit" bedeutet die zur Beladungserhöhung von 80 auf 105g/Nm³ benötigten Tage).

### Beispiel 17: Herstellung von PSA bei Beladungen bis 105g o-Xylol/Nm³-Luft (Vergleich)

Man verfährt wie unter Beispiel 12 beschrieben, mit dem Unterschied, dass durch das Rohr stündlich von oben nach unten 4,0Nm³-Luft mit Beladungen an 98,5 Gew.-%igem o-Xylol von 85 bis etwa 105/Nm-Luft geleitet wird. Dabei wurden bei 95-105g o-Xylol/Nm³ Luft die in Tabelle 2 zusammengefaßten Ergebnisse erhalten (Ausbeute bedeutet das erhaltene PSA in Gewichtsprozent, bezogen auf 100%iges o-Xylol; Lasterhöhungszeit bedeutet die zur Beladungserhöhung von 80 auf 105g/Nm³ benötigten Tage).

**Tabelle 2:**

| Beispiel: Katalysatorkombination | Salzbad-temperatur | Lasterhöhungszeit | durchschnittliche PSA-Ausbeute während 30d (Gew.-%) | durchschnittliche Phthalidgehalt im Roh-PSA während 30d (Mol-%) |
|---|---|---|---|---|
| 14: I(a)/IV(b)/ VII(c) | 375 | 13 | 110 | 0,14-0,19 |
| 15: II(a), V(b), VII (c) | 366 | 15 | 111,5 | 0,14-0,24 |
| 16:Vergleich IV (b)/VII (c) | 356-353 | Beladung kann auch über längeren Zeitraum nicht auf Werte über 90g oXylol/Nm³-Luft erhöht werden | (siehe Anmerkung bei "Lasterhöhungszeit") | (siehe Anmerkung bei "Lasterhöhungszeit") |
| 17: Vergleich I(a)/VII(c) | 370-366 | Beladung könnte zwar auf die gewünschte 105g o-Xylol/Nm³-Luft erhöht werden; ab Beladungswerten von 90g/Nm³ werden aber neben hohen Phthalidwerten signifikante Xylolmengen von 0,1-0,2% im Abgas beobachtet | (siehe Anmerkung bei "Lasterhöhungszeit") | (siehe Anmerkung bei "Lasterhöhungszeit") |

## Patentansprüche

1. Verfahren zur Herstellung von Phthalsäureanhydrid durch katalytische Gasphasenoxidation von Xylol und/oder Naphthalin mit einem molekularen Sauerstoff enthaltenden Gas in einem Festbett bei erhöhter Temperatur und mittels mindestens drei in Schichten übereinander angeordneter Schalenkatalysatoren, auf deren Kern aus Trägermaterial eine Schicht aus katalytisch aktiven Metalloxiden aufgebracht ist, **dadurch gekennzeichnet, dass** die Katalysatoraktivität von Schicht zu Schicht von der Gaseintrittsseite zur Gasaustrittsseite ansteigt, wobei die Aktivität der Katalysatoren der einzelnen Schichten so eingestellt wird, dass der am geringsten aktive Katalysator eine geringere Aktivmassengehalt und gegebenenfalls zusätzlich mehr Alkalimetall, ausgewählt aus der Gruppe bestehend aus Kalium, Rubidium und Cäsium, als Dotierung als der Katalysator der nächsten Schicht aufweist und der darauf folgende noch aktivere Katalysator die gleiche Aktivmassenmenge und noch weniger Alkalimetall als Dotierung oder eine größere Aktivmassenmenge und gegebenenfalls weniger Alkalimetall als Dotierung als der Katalysator der zweiten Schicht aufweist, mit der Maßgabe, dass
a) der am geringsten aktive Katalysator auf nicht porösem Trägermaterial 5 bis 9 Gew.-%, bezogen auf den gesamten Katalysator, Aktivmasse aufweist, enthaltend 3 bis 8 Gew.-% V₂O₅, 0 bis 3,5 Gew.-% Sb₂O₃, 0 bis 0,3 Gew.-% P, 0,1 bis 0,5 Gew.-% Alkali (ber. als Alkalimetall) und als Rest TiO₂ in Anatasform mit einer BET-Oberfläche von 18 bis 22 m²/g,
b) der nächst aktivere Katalysator bei sonst gleicher Zusammensetzung wie Katalysator (a) einen um 1 bis 5 Gew.-% (absolut) höheren Aktivmassengehalt hat und der Alkaligehalt um 0 bis 0,25 Gew.-% (absolut) geringer ist und
c) der aktivste Katalysator bei sonst gleicher Zusammensetzung wie (a) einen um 1 bis 5 Gew.-% (absolut) höheren Aktivmassengehalt als für (a) hat und der Alkaligehalt um 0,15 bis 0,4 Gew.-% (absolut) geringer als für (a) ist.

2. Verfahren nach Anspruch 1, **dadurch gekennzeichnet, dass** man Cäsium als Alkalimetall in Mengen von 0,25 bis 0,5 Gew.-% in dem am wenigstenaktiven Katalysator verwendet.

3. Verfahren nach Anspruch 1, **dadurch gekennzeichnet, dass** die Aktivität der einzelnen Katalysatoren so eingestellt wird, dass der aktivste Katalysator die gleiche oder eine größere Aktivmassenmenge und weniger Alkalimetall als Dotierung als der Katalysator der zweiten Schicht enthält.

4. Verfahren nach Anspruch 1, **dadurch gekennzeichnet, dass** die Aktivität der einzelnen Katalysatoren so eingestellt wird, dass der Katalysator der zweiten Schicht eine größere Aktivmassenmenge und weniger Alkalimetall als Dotierung als der Katalysator der ersten Schicht enthält.

5. Verfahren nach Anspruch 1, **dadurch gekennzeichnet, dass** die Aktivität der einzelnen Katalysatoren so eingestellt wird, dass der Katalysator der zweiten Schicht die gleiche Aktivmassenmenge und mehr Alkalimetall als Dotierung als der aktivste Katalysator enthält.

6. Verfahren nach Anspruch 1, **dadurch gekennzeichnet, dass** der Katalysator (a) einen Aktivmassengehalt von 6 bis 8 Gew.-% aufweist, enthaltend 4 bis 8 Gew.-% V₂O₅ und 0,3 bis 0,5 Gew.-% Cs (berechnet als Cs), der Katalysator (b) einen Aktivmassengehalt von 8 bis 12 Gew.-% aufweist, enthaltend 0,2 bis 0,5 Gew.-% Cs und dass der Katalysator (c) einen Aktivmassengehalt von 8 bis 12 Gew.-% aufweist, enthaltend 0 bis 0,3 Gew.-% Cs.

7. Verfahren nach Anspruch 1, **dadurch gekennzeichnet, dass** der Katalysator (a) einen Aktivmassengehalt von 7 bis 8 Gew.-% aufweist, enthaltend 6 bis 8 Gew.-% V₂O₅ und 0,3 bis 0,4 Gew.-% Cs, der Katalysator (b) einen Aktivmassengehalt von 9 bis 11 Gew.-% aufweist, enthaltend 0,2 bis 0,4 Gew.-% Cs und dass der Katalysator (c) einen Aktivmassengehalt von 9 bis 11 Gew.-% aufweist, enthaltend 0,05 bis 0,2 Gew.-% Cs.

## Claims

1. A process for preparing phthalic anhydride by catalytic gas-phase oxidation of xylene and/or naphthalene by a gas comprising molecular oxygen in a fixed bed at elevated temperature and using at least three coated catalysts arranged in superposed zones, which catalysts have a layer of catalytically active metal oxides applied to a core of support material, wherein the catalyst activity rises from zone to zone from the gas inlet end to the gas outlet end and the activity of the catalysts of the individual zones is set such that the least active catalyst comprises a lower amount of active composition and, if desired, additionally more alkali metal selected from the group consisting of potassium, rubidium and cesium as dopant than the catalyst of the next zone and the subsequent even more active catalyst comprises the same amount of active composition and even less alkali metal as dopant or a greater amount of active composition and, if desired, less alkali metal as dopant than the catalyst of the second zone, with the proviso that
a) the least active catalyst on nonporous support material comprises from 5 to 9% by weight, based on the total catalyst, of active composition comprising from 3 to 8% by weight of V₂O₅, from 0 to 3.5% by weight of Sb₂O₃, from 0 to 0.3% by weight of P, from 0.1 to 0.5% by weight of alkali metal (calculated as metal) and as balance TiO₂ in anatase form having a BET surface area of from 18 to 22 m²/g,
b) the next more active catalyst has the same composition as catalyst (a) except for an active composition content which is from 1 to 5% by weight (absolute) higher and an alkali metal content which is from 0 to 0.25% by weight (absolute) lower and
c) the most active catalyst has the same composition as (a) except for an active composition content which is from 1 to 5% by weight (absolute) higher than in (a) and an alkali metal content which is from 0.15 to 0.4% by weight (absolute) lower than in (a).

2. A process as claimed in claim 1, wherein cesium is used in amounts of from 0.25 to 0.5% by weight as alkali metal in the least active catalyst.

3. A process as claimed in claim 1, wherein the activity of the individual catalysts is set such that the most active catalyst comprises the same amount or a greater amount of active composition and less alkali metal as dopant than the catalyst of the second zone.

4. A process as claimed in claim 1, wherein the activity of the individual catalysts is set such that the catalyst of the second zone comprises a greater amount of active composition and less alkali metal as dopant than the catalyst of the first zone.

5. A process as claimed in claim 1, wherein the activity of the individual catalysts is set such that the catalyst of the second zone comprises the same amount of active composition and more alkali metal as dopant than the most active catalyst.

6. A process as claimed in claim 1, wherein the catalyst (a) has an active composition content of from 6 to 8% by weight comprising from 4 to 8% by weight of V₂O₅ and from 0.3 to 0.5% by weight of Cs (calculated as Cs), the catalyst (b) has an active composition content of from 8 to 12% by weight comprising from 0.2 to 0.5% by weight of Cs and the catalyst (c) has an active composition content of from 8 to 12% by weight comprising from 0 to 0.3% by weight of Cs.

7. A process as claimed in claim 1, wherein the catalyst (a) has an active composition content of from 7 to 8% by weight comprising from 6 to 8% by weight of V₂O₅ and from 0.3 to 0.4% by weight of Cs, the catalyst (b) has an active composition content of from 9 to 11% by weight comprising from 0.2 to 0.4% by weight of Cs and the catalyst (c) has an active composition content of from 9 to 11% by weight comprising from 0.05 to 0.2% by weight of Cs.

## Revendications

1. Procédé de production d'anhydride phtalique par oxydation catalytique en phase gazeuse de xylène et/ou naphtaline avec un gaz contenant de l'oxygène moléculaire dans un lit fixe à température élevée et au moyen d'au moins trois catalyseurs en coquille disposés en couches superposées, une couche d'oxydes métalliques ayant une activité catalytique étant déposée sur leur noyau composé de matière support, **caractérisé en ce que** l'activité des catalyseurs augmente d'une couche à l'autre du côté entrée du gaz jusqu'au côté de sortie du gaz, dans lequel l'activité des catalyseurs des diverses couches est ajustée de façon que le catalyseur le plus faiblement actif présente une plus faible teneur en masse active et, éventuellement, plus de métal alcalin, choisi dans le groupe consistant en potassium, rubidium et césium, en dotation que le catalyseur de la couche suivante et que le catalyseur suivant encore plus actif présente la même quantité de masse active et encore moins de métal alcalin en dotation ou une plus grande quantité de masse active et, éventuellement, moins de métal alcalin en dotation que le catalyseur de la deuxième couche, à la condition que
a) le catalyseur le plus faiblement actif présente sur matière support non poreuse 5 à 9 % en poids, en fonction du catalyseur total, de masse active, contenant 3 à 8 % en poids de V₂O₅, 0 À 3,5 % en poids de Sb₂O₃, 0 à 0,3 % en poids de P, 0,1 à 0,5 % en poids d'alcalins (calculés en tant que métal alcalin) et, comme reste, TiO₂ sous forme anatase avec une surface BET de 18 à 22 m²/g,
b) le catalyseur plus actif suivant, ayant autrement la même composition que le catalyseur (a), présente une teneur en masse active de 1 à 5 % en poids (dans l'absolu) plus élevée et la teneur en alcalins est de 0 à 0,25 % en poids (dans l'absolu) plus faible, et
c) le catalyseur le plus actif, ayant autrement la même composition que (a), a une teneur en masse active de 1 à 5 % en poids (dans l'absolu) plus élevée que pour (a) et la teneur en alcalins est de 0,15 à 0,4 % en poids (dans l'absolu) plus faible que pour (a).

2. Procédé selon la revendication 1, **caractérisé en ce que** l'on utilise du césium comme métal alcalin dans des quantités de 0,25 à 0,5 % en poids dans le catalyseur le moins actif.

3. Procédé selon la revendication 1, **caractérisé en ce que** l'activité des divers catalyseurs est ajustée de façon que le catalyseur le plus actif contient une quantité de masse active qui est la même ou plus grande et moins de métal alcalin en dotation que le catalyseur de la deuxième couche.

4. Procédé selon la revendication 1, **caractérisé en ce que** l'activité des divers catalyseurs est ajustée de façon que le catalyseur de la deuxième couche contient une quantité de masse active plus grande et moins de métal alcalin en dotation que le catalyseur de la première couche.

5. Procédé selon la revendication 1, **caractérisé en ce que** l'activité des divers catalyseurs est ajustée de façon que le catalyseur de la deuxième couche contient la même quantité de masse active et plus de métal alcalin en dotation que le catalyseur le plus actif.

6. Procédé selon la revendication 1, **caractérisé en ce que** le catalyseur (a) présente une teneur en masse active de 6 à 8 % en poids, contenant 4 à 8 % en poids de V₂O₅ et 0,3 à 0,5 % en poids de Cs (calculé en tant que Cs), le catalyseur (b) présente une teneur en masse active de 8 à 12 % en poids, contenant 0,2 à 0,5 % en poids de Cs et **en ce que** le catalyseur (c) présente une teneur en masse active de 8 à 12 % en poids, contenant 0 à 0,3 % en poids de Cs.

7. Procédé selon la revendication 1, **caractérisé en ce que** le catalyseur (a) présente une teneur en masse active de 7 à 8 % en poids, contenant 6 à 8 % en poids de V₂O₅ et 0,3 à 0,4 % en poids de Cs, le catalyseur (b) présente une teneur en masse active de 9 à 11 % en poids, contenant 0,2 à 0,4 % en poids de Cs et **en ce que** le catalyseur (c) présente une teneur en masse active de 9 à 11 % en poids, contenant 0,05 à 0,2 % en poids de Cs.
